(19) Europäisches Patentamt / European Patent Office / Office européen des brevets

(11) **EP 4 712 004 A1**

(12) **EUROPEAN PATENT APPLICATION**
published in accordance with Art. 153(4) EPC

(43) Date of publication:
**18.03.2026 Bulletin 2026/12**

(21) Application number: **24803758.2**

(22) Date of filing: **09.05.2024**

(51) International Patent Classification (IPC):
**G06Q 30/06** *(2023.01)*    **G06Q 50/04** *(2012.01)*
**G06Q 30/02** *(2023.01)*    **G16H 10/20** *(2018.01)*
**A61B 5/00** *(2006.01)*    **G06Q 30/0601** *(2023.01)*
**G16H 20/00** *(2018.01)*    **G16H 30/40** *(2018.01)*
**G16H 50/20** *(2018.01)*

(52) Cooperative Patent Classification (CPC):
**G06Q 50/04; A61B 5/00; G06Q 30/02; G06Q 30/06;
G06Q 30/0621; G06Q 30/0631; G16H 10/20;
G16H 20/60; G16H 30/40; G16H 40/63;
G16H 40/67; G16H 50/20; G16H 50/30;
G16H 50/70**

(86) International application number:
**PCT/KR2024/006295**

(87) International publication number:
**WO 2024/232705 (14.11.2024 Gazette 2024/46)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **10.05.2023 KR 20230060680
03.05.2024 KR 20240058975**

(71) Applicant: **Lillycover Co., Ltd.
Daegu 41256 (KR)**

(72) Inventor: **AN, Sun Hee
Gyeongsan-si, Gyeongsangbuk-do 38659 (KR)**

(74) Representative: **M. Zardi & Co S.A.
Via G. B. Pioda, 6
6900 Lugano (CH)**

(54) **METHOD FOR PROVIDING CUSTOMIZED PRODUCT**

(57) The present invention provides a method for providing a customized product, comprising the steps of: collecting, from a user, survey response information and an image in response to survey request information and image request information provided to the user in order to diagnose at least one of the skin, scalp, health condition, and preference of the user; generating a diagnosis result for at least one of the skin, scalp, health condition, and preference of the user on the basis of the survey response information and the image; generating and providing recipe information for manufacturing a user-customized product with respect to the diagnosis result; and transmitting, to a product manufacturing device, a user-customized product manufacturing instruction signal including the recipe information on the basis of a user-customized product purchase signal received from the user, wherein the recipe information includes a raw material recommended by a diagnosis code including the diagnosis result and a raw material code including raw material information.

FIG.1

## Description

### TECHNICAL FIELD

The present invention relates to a method for providing a user-customized product, which grasps information on a user and provides a product to the user to be suitable for the grasped information.

### BACKGROUND ART

As the attention has shifted from the mass of people to individuals recently with the improvement in the living standards, and quality of life of individuals is recognized as an important factor, desire of individuals to pursue a beautiful and healthy life is increasing. Accordingly, investment in customized products, such as cosmetics, health functional foods, and preference items, considering the tastes, preferences, conditions, and the like of individuals is increasing significantly.

Cosmetics are used in a way of applying, rubbing, or spraying onto the body to clean and beautify the body to enhance attractiveness and change the appearance to be brightened, or to maintain or improve health of the skin and hair. Accordingly, as the cosmetics are directly applied to human bodies, particularly to the skin and scalp, it is important to grasp the condition of the skin and scalp of each individual. In addition, as appropriate raw materials and/or dosage forms are selected on the basis of the grasped information, the effect can be maximized, and side effects such as skin troubles can be minimized.

Meanwhile, health functional foods are foods manufactured and processed using raw materials or ingredients having functionalities beneficial to human bodies, and may obtain beneficial effects in adjusting nutrients for the structure and/or function of human body or in the physiological actions. The health functional foods may have beneficial effects on the nervous system, sensory system, digestive/metabolic system, cardiovascular system, reproductive system, urinary system, muscular system, body defense and immune system, children's growth, sperm motility, and the like. Although the health functional foods are distinguished from general foods and medicines and helpful in maintaining the health, there are concerns about misuse, side effects, and reduction in the effect when they are consumed without considering health conditions of individuals and currently taking medicines or health functional foods.

In addition, preference items, among the foods or objects, are not the foods consumed for survival, but are used habitually to feel the taste or scent. Particularly, preference foods are not the foods that should be essentially taken to sustain life, but are foods taken to satisfy psychological needs through the taste and scent. Therefore, the preference foods are consumed only to enjoy the taste and scent regardless of the type and content of nutrients. The types of preference items are various, such as cigarettes, alcohol, coffee, soft drinks, snacks, seasoned foods, and the like, and representative preference items are cigarettes and/or coffee.

Meanwhile, since preferences that each individual feels are different, it is important to grasp preferences of individuals and adjust the preferences, such as the taste, scent, alcohol, alkaloids (nicotine, caffeine, etc.), carbon dioxide, and the like, to be suitable for the taste of each individual.

### DISCLOSURE OF INVENTION

### TECHNICAL PROBLEM

Therefore, the present invention has been made in view of the above problems, and it is an object of the present invention to generate a result of diagnosing a user from the image and/or survey response result of the user, and manufacture and provide a user-customized product on the basis of recipe information generated according to the diagnosis result.

### TECHNICAL SOLUTION

To accomplish the above object, according to one aspect of the present invention, there is provided a method for providing a customized product, the method comprising the steps of: collecting, from a user, survey response information and an image in response to survey request information and image request information provided to the user in order to diagnose any one or more among the skin, scalp, health condition, and preference of the user; generating a result of diagnosing any one or more among the skin, scalp, health condition, and preference of the user on the basis of the survey response information and the image; generating and providing recipe information for manufacturing a user-customized product with respect to the diagnosis result; and transmitting, to a product manufacturing device, a user-customized product manufacturing instruction signal including the recipe information on the basis of a user-customized product purchase signal received from the user, wherein the recipe information includes a raw material recommended by a diagnosis code including the diagnosis result and a raw material code including raw material information.

## ADVANTAGEOUS EFFECTS

As the method for providing a customized product of the present invention may generate a result of diagnosing the skin of a user from the image and/or survey response result of the user, and manufacture and provide a user-customized skin product on the basis of recipe information generated according to the diagnosis result, it will be helpful for skin care of the user.

In addition, as the method may generate a result of diagnosing the scalp of a user from the image and/or survey response result of the user, and manufacture and provide a user-customized scalp product on the basis of recipe information generated according to the diagnosis result, it will be helpful for scalp care of the user.

In addition, as a result of diagnosing the scalp of a user is determined and provided as any one among a plurality of scalp types classified in the form of MBTI, the user may intuitively recognize the scalp condition of the user and easily learn the method of caring according thereto.

In addition, a result of diagnosing the health condition and/or preference of a user is generated from the image and/or survey response result of the user, and a user-customized product is manufactured and provided on the basis of recipe information generated according to the diagnosis result, it will be helpful to maintain the health and/or satisfy the preference of the user.

In addition, as user-customized skin products, scalp products, health functional foods, and/or preference items are manufactured using a product manufacturing device that receives a request for purchasing the skin products, scalp products, health functional foods, and/or preference items from a user on the basis of recipe information generated according to the diagnosis result, satisfaction of the user can be enhanced.

Although the skin products, scalp products, health functional foods, and/or preference items themselves are products produced massively and uniformly, as a user perceives the skin products, scalp products, health functional foods, and/or preference items as personalized products, satisfaction of the user with the products can be enhanced.

The effects of the present invention are not limited to the effects mentioned above, and unmentioned other effects will be clearly understood by those skilled in the art from the following description.

## BRIEF DESCRIPTION OF THE DRAWINGS

FIG. 1 is a view showing an example of a network environment according to an embodiment of the present invention.

FIG. 2 is a block diagram schematically showing the configuration of a device according to an embodiment of the present invention.

FIG. 3 is a block diagram schematically showing the configuration of a service provision management unit of the device of FIG. 2.

FIG. 4 is an exemplary view schematically showing an example of a skin diagnosis result of a customer according to an embodiment of the present invention.

FIG. 5 is an exemplary view schematically showing an example of a table of scalp diagnosis criteria expressed in the form of MBTI for generating a scalp diagnosis result of a customer according to an embodiment of the present invention.

FIG. 6a and FIG. 6b are tables schematically showing an example of storing raw material information according to an embodiment of the present invention.

FIG. 7 is a block diagram schematically showing the configuration of a device according to another embodiment of the present invention.

FIG. 8 is a flowchart illustrating a method for providing a user-customized product according to an embodiment of the present invention.

FIG. 9 is a flowchart illustrating a method for providing a user-customized product according to another embodiment of the present invention.

## BEST MODE FOR CARRYING OUT THE INVENTION

In order to accomplish the object described above, an embodiment of the present invention provides a method for providing a customized product, the method comprising the steps of: collecting, from a user, survey response information and an image in response to survey request information and image request information provided to the user in order to diagnose any one or more among the skin, scalp, health condition, and preference of the user; generating a result of diagnosing any one or more among the skin, scalp, health condition, and preference of the user on the basis of the survey response information and the image; generating and providing recipe information for manufacturing a user-customized product with respect to the diagnosis result; and transmitting, to a product manufacturing device, a user-customized product manufacturing instruction signal including the recipe information on the basis of a user-customized product purchase signal received from the user, wherein the recipe information includes a raw material recommended by a diagnosis code including the diagnosis result and a raw material code including raw material information.

The raw material code may further include a raw material purity score and a raw material proportion score, and the recipe information may provide a score calculated by a sum of the raw material purity score and the raw material proportion score.

The recipe information may include a raw material mixing ratio, wherein the raw material mixing ratio is calculated using artificial intelligence that has learned to output an amount of required raw materials.

The step of generating and providing recipe information for manufacturing a user-customized product with respect to the diagnosis result may further include the step of recommending purchase of a user-customized product corresponding to the recipe information, where a product recommended at the step of recommending purchase of a user-customized product may be determined by equation 1.

[Equation 1]

$$\frac{1}{3}\left[\left(\frac{F_p+P_p+W_p+C_p+W_p+O_p}{6} \times 0.56\right) + (PS \times 0.34) + (PSd \times 0.1)\right]$$

(Here, Fp: flush percentage, Pp: pore percentage, Wp: wrinkle percentage, Cp: pigment percentage, Op: oil and moisture percentage, Sp: sensitivity percentage, PS: purchase satisfaction survey value, PSd: big data label purchase satisfaction survey value)

The image may be transmitted to an artificial intelligence service, and the diagnosis result may be generated using a result generated by analysis of the artificial intelligence.

The method for providing a customized product may further comprise, after the step of generating and providing recipe information, the step of generating a user-customized product label using the recipe information, wherein a space where product information and user's order information are displayed may be provided on the label, and areas other than the space where product information and user's order information are displayed may be freely customized by the user.

Before describing the preferred embodiments of the present invention below in detail, it should be noted that the terms and words used in this specification and claims should not be interpreted as being limited to their usual or dictionary meanings, but should be interpreted as meanings and concepts corresponding to the technical spirit of the present invention.

Hereinafter, embodiments of the present invention will be illustrated in the drawings and described in detail in the detailed description. The effects and/or features of the present invention and the methods for achieving them will become clear with reference to the embodiments described below in detail together with the drawings. However, the present invention is not limited to the embodiments disclosed below and may be implemented in various forms.

Hereinafter, the embodiments of the present invention will be described in detail with reference to the accompanying drawings. In describing with reference to the drawings, the same reference numerals are given to identical or corresponding components, and redundant descriptions thereof will be omitted.

In the embodiments described below, the terms such as first, second, and the like are not used in a limiting sense, but are used for the purpose of distinguishing one component from another.

In the embodiments described below, singular expressions include plural expressions unless the context clearly indicates otherwise.

In the embodiments described below, the terms such as "include", "have", and the like mean existence of features or components described in the specification, and do not exclude in advance the possibility that one or more other features or components may be added.

For the convenience of explanation, the size of components in the drawings may be exaggerated or reduced. For example, as the size and/or thickness of each component shown in the drawings are arbitrarily shown for the convenience of explanation, the present invention is not necessarily limited to those shown in the drawings.

In the embodiments described below, the x-axis, y-axis, and/or z-axis are not limited to the three axes on the orthogonal coordinate system, and may be interpreted in a broad sense including the same. For example, although the x-axis, y-axis, and/or z-axis may be orthogonal to each other, they may also refer to different directions that are not orthogonal to each other.

In the case where an embodiment can be implemented in a different way, a specific processing order may be performed in an order different from those described in the embodiment. For example, two successively described processes may be performed at the same time practically, or may be progressed in an order reverse to the described order.

In addition, in the present application, a "unit" may be a hardware component such as a processor or a circuit and/or a software component executed by a hardware component such as a processor.

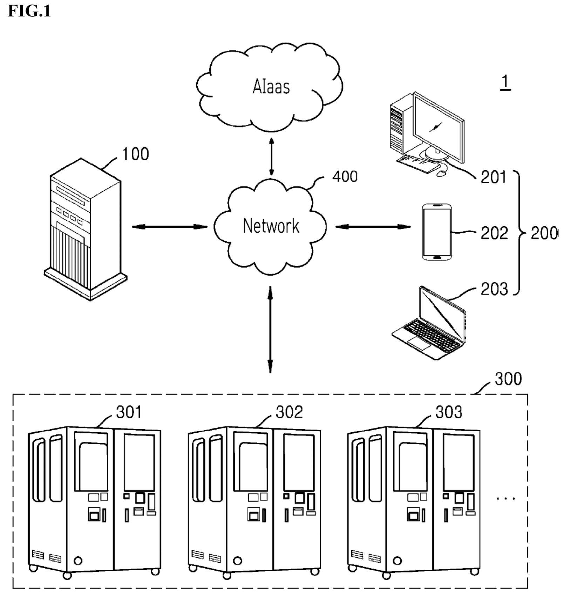

An embodiment of the present invention relates to a method for providing a customized product, and is described with reference to FIG. 1, which summarizes the sequence of the manufacturing method. According to this embodiment, as the method for providing a user-customized product generates a result of diagnosing the skin and scalp of a user from the image and/or survey response result of the user, and manufactures and provides a user-customized skin product on the basis of recipe information generated according to the diagnosis result, it will be helpful for caring the skin and scalp of the user. In addition, as a result of diagnosing the scalp of a user is determined and provided as any one among a plurality of scalp types classified in the form of MBTI, the user may intuitively recognize the scalp condition of the user and easily learn the method of caring according thereto. In addition, as user-customized skin products, scalp products, health functional foods, and/or preference items are manufactured using a product manufacturing device that receives a request for purchasing the skin products, scalp products, health functional foods, and/or preference items from a user on the basis of recipe information generated according to the diagnosis result, satisfaction of the user can be enhanced.

A user-customized provision device 100 according to an embodiment of the present invention may transmit survey request information and/or image request information to a user terminal 200 connected through a network 400 to diagnose any one or more among the skin, scalp, health condition, and/or preference of the user.

For example, the survey request information may be questions that can determine any one or more among the skin, scalp, health condition, and/or preference of the user, and include, for example, the number of times of washing the face (e.g., twice a day), the degree of skin tightness before and after washing the face, the types of skin products currently in use (e.g., cleanser, basic cosmetics, color cosmetics, packs, etc.) and/or the feeling after using the same, the number of times of washing the hair (e.g., once a day), the degree of scalp tightness after washing the hair, the types of hair products currently in use (shampoo, conditioner, treatment, etc.) and/or the feeling after using the same, the types and/or ingredients of health functional foods currently taking (EPA and/or DHA containing oil, ginkgo leaf extract, red ginseng, BT-11 polygala extract powder, wolfberry extract, angelica root extract complex, loquat leaf extract, lactic acid bacteria fermented kelp extract, sesame seed extract, Gastrodia elata complex extract, grape and blueberry extract mixed powder (Memophenol TM), fibroin extract BF-7, milk protein hydrolysate, theanine, wild ginseng leaf extract, ashwagandha extract, L-glutamic acid fermented GABA powder, kelp extract, rice bran ethanol extract, Hydrolysed Casein Protein, phosphatidylserine, Angelica Gigas Extract Powder, Angelica Gigas Root Extract, Prunus Mume Fruit Extract, ginseng, rhodiola extract, Fermented Porcine Placenta Extract, fermentation-produced amino acid complex, Hovenia Dulcis Fruit Extract Powder, Calendula Officinalis Flower Extract, bilberry extract, Haematococcus Pluvialis extract, persimmon leaf ethanol extract powder, Vaccinium uliginosum extract, lutein/zeaxanthin complex extract, zeaxanthin extract, Chazugi extract, Enzymatically Fermented Grape Skin Extract (KL-GEFE), xylitol, NAG (N-Acetylglucosamine), konjac potato extract, chlorophyll-containing plants, Chlorella, hyaluronic acid, AP Collagen Hydrolyzed Peptide, Collagen peptide, PME-88 melon extract, Galactooligosaccharide powder (Neogose-P70), rosemary grapefruit extract complex (Nutroxsun), rosemary extract complex (SunMax), dandelion extract complex, wheat extract (Ceratiq®), wheat germ oil extract, perilla extract (Agatri®), pomegranate concentrate powder, pomegranate concentrate, pine bark extract complex, Hydrangea leaf hydrothermal extract, Hydrangea leaf hydrothermal extract, rice bran extract, corn germ extract, low molecular collagen peptide, low molecular collagen peptide GT, low molecular collagen peptide NS, low molecular collagen peptide SH, Lithospermum Officinale Extract , soybean and barley fermented complex, probiotics, fingerroot extract powder, Honeybush extract fermented powder, milk thistle extract, Partially Hydrolyzed Guar Gum, glucomannan, indigestible maltodextrin, soy dietary fiber, raffinose, wood ear mushroom dietary fiber, wheat dietary fiber, barley dietary fiber, powdered agar, gum arabic (acacia gum), aloe gel, aloe whole leaf, inulin/chicory extract, psyllium husk dietary fiber, polydextrose, Fructooligosaccharides, probiotics, garcinia cambogia extract, Conjugated linoleic acid, green tea extract, chitosan/Chito-oligosaccharide, poly-gamma-glutamic acid, Gamma-Linolenic Acid (GLA) containing oil, lecithin, garlic, policosanol, coenzyme Q10, saw palmetto fruit extract, mucopolysaccharide protein, soy isoflavone, MSM (methyl sulfonylmethane, dimethyl sulfone), L-carnitine tartrate, parkran Cranberry powder, pumpkin seed extract, Phellinus Linteus Extract, shark liver oil containing alkoxyglycerol, squalene, propolis extract, etc.), blood pressure, diabetes, in-body information (weight, skeletal muscle mass, mineral mass, etc.), eyesight, diet, eating habits, bowel habits, stress, sleeping hours, menstrual cramps, premenstrual syndrome (PMS), exercise habits, preferred foods, preferred tastes, preferred scents, preferred tobacco products, amount of smoking, amount of alcohol consumed, and the like.

In addition, the image request information is a plurality of request images capable of determining one or more among the skin conditions and/or scalp conditions of the user, and may include front and/or side images of the face of the user, an image of the T-zone where sebum is concentrated, an image of the hairline of the user, an image of the scalp of the user, and the like.

As an optional embodiment, the user-customized provision device 100 may provide photograph setting information to the user terminal 200 in response to the specification information of the user terminal 200. This may be provided for optimal image collection, as the camera provided in each user terminal 200 is different. Here, the photograph setting level may include brightness, sensitivity, contrast, focus, photographing composition guide information, and the like.

The user-customized provision device 100 may collect survey response information and/or captured images from the user terminal 200 in response to the survey request information and/or image request information.

The user-customized provision device 100 may generate any one or more among a result of diagnosing the skin of the user, a result of diagnosing the scalp of the user, a result of diagnosing the health condition of the user, and a result of diagnosing the preference of the user on the basis of the survey response information and/or image, and provide the diagnosis result to the user terminal 200.

For example, the user-customized provision device 100 may generate a result of diagnosing skin items including oil and moisture, sensitivity, pigmentation, wrinkles, flush, and/or pores of the skin of the user as a skin diagnosis result. In addition, the user-customized provision device 100 may generate a result of diagnosing scalp items including oil and moisture, sensitivity, hair thickness, hair damage, hair loss, and/or keratin for the scalp of the user as a scalp diagnosis result, and may determine the scalp diagnosis result as any one among a plurality of scalp types classified in the form of MBTI.

In addition, the user-customized provision device 100 may generate a result of diagnosing health items including the blood pressure, heart rate, electrocardiogram, stress index, fatigue, and body parts requiring treatment (nervous system, sensory system, digestive/metabolic system, cardiovascular system, endocrine system, reproductive system, urinary system, muscular system, immune system, etc.) of the user, and/or the types of required nutrients as a health diagnosis result, and the user-customized provision device 100 may generate a result of diagnosing preference items including any one or more among the taste, scent, content of nicotine, and content of caffeine selected according to the preference items (cigarettes, alcohol, coffee, etc.) or perfumes preferred by the user as a preference diagnosis result.

The user-customized provision device 100 may provide the user terminal 200 with a solution that may solve the problems related to one or more of the diagnosis results described above.

As an optional embodiment, the solution may include a primary solution, a secondary solution, and/or a tertiary solution.

The primary solution may provide a visual image and a score of one or more of the skin diagnosis results described above at the time point of past and/or present, and may include comments that may solve the problem.

The secondary solution may provide comments on treatment, a treatment method, and images comparing the results before/after the treatment when the treatment method is performed. For example, the secondary solution may provide a skin massage method and images comparing the results before/after the skin massage when the skin massage is performed using the method. In addition, the secondary solution may include product recipe information for providing a user-customized product for any one or more among the skin diagnosis result, scalp diagnosis result, health diagnosis result, and/or preference diagnosis result.

The tertiary solution may include a non-face-to-face coaching service provided to the user to solve the problems related to any one or more among the diagnosis results described above. The non-face-to-face coaching service matches an appropriate expert with each user, and at this point, the expert is, for example, a specialist, a dermatologist, a pharmacist, a perfumer, or the like. The non-face-to-face coaching service may acquire a result of diagnosing any one or more among the skin, scalp, health condition, and preference of the user generated by the matched expert, recipe information corresponding to the diagnosis result, and/or coaching data based on manufactured customized product information, and provide the coaching data to the user at a predetermined time and/or cycle. In the present embodiment, an expert terminal (not shown) connected the user-customized provision device 100 through the network 400 may be further included to provide the non-face-to-face coaching service.

The user-customized provision device 100 may provide recipe information of a product corresponding to any one or more among the skin diagnosis result, scalp diagnosis result, health diagnosis result, and preference diagnosis result to the user terminal 200, and recommend purchase of a user-customized product corresponding to the recipe information.

When a user-customized product purchase signal corresponding to the recipe information is received from the user terminal 200, the user-customized provision device 100 may transmit user-customized product manufacturing information to the product manufacturing device, and direct a task of manufacturing the user-customized product. Here, the user-customized product manufacturing information may include any one or more among the recipe information, raw material mixing information, container information, and stirring information corresponding to any one or more among the skin diagnosis result, scalp diagnosis result, health diagnosis result, and preference diagnosis result of the user.

As a manufacturing completion signal for the user-customized product is received from the product manufacturing device 300, the user-customized provision device 100 may request quality inspection of the user-customized product. Here, quality inspection of the user-customized product may be performed by the product manufacturing device 300. Alternatively, an external quality assessment company may be requested to performed the quality inspection, through the product manufacturing device 300 that has received the request of the user-customized provision device 100.

As a quality inspection completion signal for the user-customized product is received from the product manufacturing device 300, the user-customized provision device 100 may request packaging of the user-customized product.

As a packaging completion signal for the user-customized product is received from the product manufacturing device 300, the user-customized provision device 100 may transmit delivery information of the user to a delivery company and request delivery of the user-customized product. The delivery company may pick up the user-customized product from the logistics warehouse of the product manufacturing device, and deliver the product to the user.

As an optional embodiment, before instructing the product manufacturing device 300 to perform a task, the user-customized provision device 100 may transmit inspection request information including raw material inventory request information, container inventory request information, state request information, and manufacturing environment request information to each of a plurality of product manufacturing devices 301, 302, 303. The user-customized provision device 100 may receive inspection response information from each of the plurality of product manufacturing devices 301, 302, 303 in response to the inspection request information. The user-customized provision device 100 may select at least one product manufacturing device 300 capable of manufacturing the user-customized product on the basis of the inspection response information.

The user-customized provision device 100 may exist independently in the form of a server, or may be mounted on the user terminal 200 by implementing the functions provided by the user-customized provision device 100 in the form of an application.

The user terminal 200 may access a user-customized service providing application and/or a user-customized service providing site provided by the user-customized provision device 100, and receive a user-customized service.

The user terminal 200 may include a communication terminal capable of performing the functions of a computing device, and may be, but not limited to, a desktop computer, a smart phone, a laptop computer, a tablet PC, a smart TV, a cellular phone, a personal digital assistant (PDA), a media player, a micro server, a global positioning system (GPS) device, an e-book reader, a digital broadcasting terminal, a navigation device, a kiosk, an MP3 player, a digital camera, a home appliance, and/or other mobile or non-mobile computing devices operated by a user. In addition, the user terminal 200 may be a wearable terminal such as a watch, glasses, a hair band, and/or a ring having a communication function and/or a data processing function. The user terminal 200 is not limited to those described above, and a terminal capable of web browsing may be used without limitation.

The product manufacturing device 300 may receive a product manufacturing instruction signal and/or user-customized product manufacturing information from the user-customized provision device 100, and manufacture any one or more among a user-customized skin product, a user-customized scalp product, a user-customized health functional food, and a user-customized preference item.

The product manufacturing device 300 may generate inspection response information including raw material inventory response information, container inventory response information, state response information, and/or manufacturing environment response information in response to the inspection request information received from the user-customized provision device 100, and transmit the generated inspection response information to the user-customized provision device 100. Here, the state response information may include whether the product manufacturing device is operating normally, the number of production orders, the number of manufactured products, the defect rate, the worker, the manufacturing progress location (e.g., container insertion, container separation, raw material discharge, container assembly, stirring, etc.), raw material discharge amount, raw material expiry date, raw material order state, raw material purchase and stocking state, and the like. In addition, the manufacturing environment response information may include the temperature and/or humidity inside the product manufacturing device 300, and the cleaning state inside thereof.

The product manufacturing device may perform quality inspection on the user-customized product in response to a quality inspection request signal from the user-customized provision device 100, or may request an external quality assessment company (not shown) to perform quality inspection. The product manufacturing device may transmit a result of performing quality inspection on the user-customized product to the user-customized provision device 100.

The product manufacturing device 300 may perform packaging of the user-customized product in response to a packaging request signal for the user-customized product.

The network 400 may perform a function of connecting the user-customized provision device 100, the user terminal 200, and/or the product manufacturing device. The network 400 may include, for example, wired networks such as a local area network (LAN), a wide area network (WAN), a metropolitan area network (MAN), and an integrated service digital network (ISDN), or wireless networks such as a wireless LAN (WLAN), code-division multiple access (CDMA), and satellite communication, but the scope of the present invention is not limited thereto. In addition, the network 400 may transmit and receive information using short-range communication and/or long-range communication. Here, the short-range communication may include techniques such as Bluetooth, radio frequency identification (RFID), infrared data association (IrDA), ultra-wideband (UWB), ZigBee, and Wi-Fi, and the long-range communication may include techniques such as code-division multiple access (CDMA), frequency-division multiple access (FDMA), time-division multiple access (TDMA), orthogonal frequency-division multiple access (OFDMA), and single carrier frequency-division multiple access (SC-FDMA).

The network 400 may include connections of network elements such as hubs, bridges, routers, and switches. The network 400 may include one or more connected networks, such as a multi-network environment, including public networks such as the Internet and/or private networks such as a secure corporate private network. Access through the network 400 may be provided through one or more wired or wireless access networks.

Furthermore, the network 400 may support techniques of controller area network (CAN) communication, vehicle-to-infrastructure (V2I) communication, vehicle-to-everything (V2X) communication, and wireless access in vehicular environment (WAVE) communication, and Internet of Things (IoT) network and/or 5G communication that exchange and process information between distributed components such as objects.

In addition, the user-customized provision device 100 may also be connected a cloud-based AI-as-a-service (Alaas) provided by an AI service company through the network 400. When connected to the AI-as-a-service (Alaas), as AI techniques can be applied, coaching data based on information such as any one or more among the skin diagnosis result, scalp diagnosis result, health diagnosis result, and preference diagnosis result generated by the AI, product recipe information for any one or more among the skin diagnosis result, scalp diagnosis result, health diagnosis result, and preference diagnosis result, information on manufactured cosmetics, information on skin history, information on skin care, and/or nutrition facts of health functional foods and how to take the foods, information on manufactured preference items, and information on manufactured perfumes, and the like may be obtained and provided to the user terminal 200. The provision unit may provide the coaching data generated by the AI to the user terminal 200 at a predetermined time and/or cycle.

For example, the artificial intelligence (AI) may include any one or more selected from machine learning, deep learning, generative AI, multimodality, natural language processing (NLP), on-device AI, and/or artificial general intelligence (AGI).

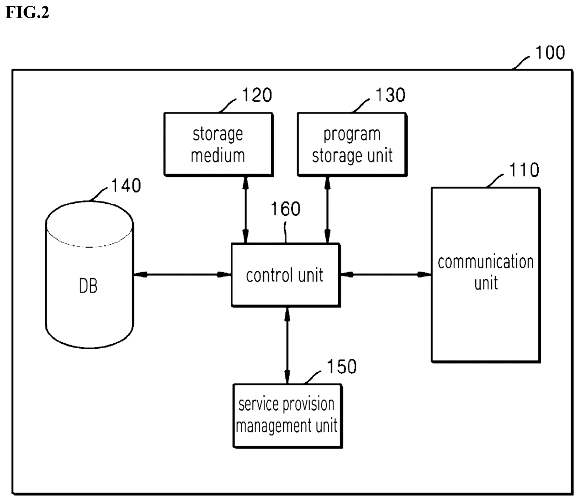

FIG. 2 is a block diagram schematically showing the configuration of a device according to an embodiment of the present invention. In the following description, any part overlapped with the description of FIG. 1 will be omitted.

Referring to FIG. 2, the user-customized provision device 100 may include a communication unit 110, a storage medium 120, a program storage unit 130, a database 140, a service provision management unit 150, and/or a control unit 160.

The communication unit 110 may provide a communication interface needed to provide transmission and reception signals in the form of packet data between the user-customized provision device 100, the user terminal 200, and/or the product manufacturing device in connection with the network 400. Furthermore, the communication unit 110 may perform a function of transmitting a predetermined information request signal processed by the service provision management unit 150 to the user terminal 200 and/or the product manufacturing device 300, and may perform a function of receiving a predetermined information response signal from the user terminal 200 and/or the product manufacturing device 300. Here, the communication network is a medium that performs a function of connecting the user-customized provision device 100, the user terminal 200, and the product manufacturing device 300, and may include a path that provides a connection path so that the user terminal 200 and/or the product manufacturing device 300 may transmit and receive information after connecting to the user-customized provision device 100. In addition, the communication unit 110 may be a device including hardware and/or software needed to transmit and receive signals such as control signals or data signals through wired or wireless connections with other network 400 devices.

The storage medium 120 performs a function of temporarily or permanently storing data processed by the control unit 160. Here, the storage medium 120 may include a magnetic storage medium or a flash storage medium, but it is not limited thereto. Such a storage medium may include a built-in memory and/or an external memory, and may include volatile memory such as DRAM, SRAM, SDRAM, and the like, non-volatile memory such as one-time programmable ROM (OTPROM), PROM, EPROM, EEPROM, mask ROM, flash ROM, NAND flash memory, and NOR flash memory, and the like, a flash drive such as SSD, compact flash (CF) card, SD card, Micro-SD card, Mini-SD card, Xd card, a memory stick, and the like, and a storage device such as HDD.

The program storage unit 130 is equipped with control software that performs the tasks of transmitting survey request information and/or image request information to the user terminal 200 to diagnose any one or more among the skin, scalp, health, and/or preference of the user, collecting survey response information and/or image from the user terminal 200, generating and providing a result of diagnosing any one or more among the skin, scalp, health, and/or preference of the user on the basis of the survey response information and/or image, generating and providing a solution for any one or more among the skin, scalp, health, and/or preference of the user in response to the diagnosis result, recommending purchase of a product manufactured in correspondence to recipe information included in the solution, transmitting inspection request information to each of a plurality of product manufacturing devices 301, 302, 303 in response to a product purchase request signal, receiving inspection response information in response to the inspection request information from each of the plurality of product manufacturing devices 301, 302, 303, selecting at least one product manufacturing device 300 capable of manufacturing a user-customized product on the basis of the inspection response information, transmitting user-customized product manufacturing information to the selected product manufacturing device 300 and directing a product manufacturing task, requesting quality inspection of the user-customized product as a manufacturing completion signal for the user-customized product is received from the product manufacturing device 300, requesting the product manufacturing device 300 to package the user-customized product from, and transmitting delivery information of the user to a delivery company and requesting delivery of the user-customized product, and the like.

The database 140 may include a management database for storing various information for manufacturing user-customized products. For example, the management database may store recipe information for skin products, scalp products, health functional foods, preference items, and/or perfumes, and product manufacturing information including raw material mixing information, container information, and stirring information. In addition, information on the nutrients contained in the health functional foods and/or medicines, dosages, how to take medicines, and interactions between the nutrition facts and medicines may be stored.

The management database 140 may store information on raw materials for manufacturing user-customized products. For example, information on raw materials may include certification information such as certification of the Food and Drug Administration

(FDA) of the United States and/or certification of GMP of a country where the manufacturing facility is located. In addition, a list of raw materials may be stored on the basis of the certification information, and each of the raw materials may be linked to a diagnosis code and recommended as a recipe suitable for each diagnosis. As a specific example, a unique raw material code may be assigned to each raw material, and each raw material code may be classified and stored to correspond to each diagnosis code, and a recipe suitable for each diagnosis may be recommended on the basis of the raw material code corresponding to each diagnosis code. In addition, a raw material purity score, a raw material proportion score according to each diagnosis code, and the like may be set to the raw material and stored for recommendations according to each diagnosis code.

The management database 140 may store raw material inventory response information, container inventory response information, state response information, and/or manufacturing environment response information as inspection response information received from the product manufacturing device. In addition, the management database may store information on the result of performing quality inspection on the user-customized products.

In addition, the management database 140 may store expert profile information and coaching data generated by experts to provide non-face-to-face coaching service to the user. For example, the experts may include dermatologists, skin care specialists, pharmacists, perfumers, and the like.

In addition, the database may include a user database 140 that stores information on users who will be provided with user-customized services. Here, the user information may include basic information on the user, such as the name, affiliation, personal information, gender, age, contact information, email, address, image, and the like, information on authentication (login) of the user, such as the ID (or email) and/or password, information on access country, access location, information on the device used for access, and information related to access such as the accessed network environment.

In addition, the user database 140 may store unique information of the user, information and/or category history (e.g., diagnosis history, recipe history, purchase history, etc.) provided to the user who has accessed the user-customized service providing application or user-customized service providing site, environment setting information set by the user, information on the amount of resources used by the user, and billing and/or payment information corresponding to the amount of resources used by the user.

The service provision management unit 150 may transmit survey request information and/or image request information to the user terminal 200 to diagnose any one or more among the skin, scalp, health, and/or preference of the user. The service provision management unit 150 may collect survey response information and/or image from the user terminal 200 in response to the survey request information and/or image request information.

The service provision management unit 150 may generate any one or more among a result of diagnosing the skin of the user, a result of diagnosing the scalp of the user, a result of diagnosing the health condition of the user, and/or a result of diagnosing the preference of the user on the basis of the survey response information and/or image, and provide the diagnosis result to the user terminal 200.

The service provision management unit 150 may provide a solution for any one or more among the skin diagnosis result, scalp diagnosis result, health diagnosis result, and preference diagnosis result. Specifically, the service provision management unit 150 may generate and provide recipe information for manufacturing a user-customized product included in the solution, and recommend purchase of the user-customized product corresponding to the recipe information.

The service provision management unit 150 may transmit a manufacturing instruction signal for user-customized cosmetics, health functional foods, preference items, and/or perfumes including the recipe information to a product manufacturing device on the basis of the user-customized product purchase signal received from the user terminal 200.

The service provision management unit 150 may transmit inspection request information to each of the plurality of product manufacturing devices 301, 302, 303 in response to the product purchase request signal from the user terminal 200. The service provision management unit 150 may receive inspection response information from each of the plurality of product manufacturing devices 301, 302, 303 in response to the inspection request information. The service provision management unit 150 may select at least one product manufacturing device 300 capable of manufacturing a user-customized product on the basis of the inspection response information.

The service provision management unit 150 may transmit user-customized product manufacturing information to the selected product manufacturing device 300 and direct a task. As a manufacturing completion signal for the user-customized product is received from the product manufacturing device 300, the service provision management unit 150 may request quality inspection of the user-customized product. As a quality inspection completion signal for the user-customized product is received from the product manufacturing device 300, the service provision management unit 150 may request packaging of the user-customized product. As a packaging completion signal for the user-customized product is received from the product manufacturing device, the service provision management unit 150 may transmit delivery information of the user to a delivery company (not shown) and request delivery of the user-customized product.

The control unit 160 is a type of central processing unit and may control the entire operation of an amplifier design device 100 by driving control software stored in the program storage unit 130. The control unit 160 may include all types of devices capable of processing data, such as a processor. Here, the 'processor' may mean, for example, a data processing device embedded in hardware, having a physically structured circuit to perform a function expressed by the code or instruction included in a program. Although examples of such data processing devices embedded in hardware may include processing devices such as a microprocessor, a central processing unit (CPU), a processor core, a multiprocessor, an application-specific integrated circuit (ASIC), a field programmable gate array (FPGA), and the like, they are not limited thereto.

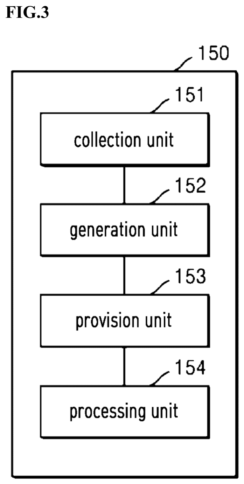

FIG. 3 is a block diagram schematically showing the configuration of the service provision management unit 150 of the user-customized provision device 100 shown in FIG. 2. In the following description, any part overlapped with the descriptions of FIGS. 1 and 2 will be omitted. Referring to FIG. 3, the service provision management unit 150 may include a collection unit 151, a generation unit 152, a provision unit 153, and/or a processing unit 154.

The collection unit 151 may collect survey response information and/or image from the user in response to survey request information and/or image request information provided to the user to diagnose any one or more among the skin, scalp, health, and/or preference of the user.

As an optional embodiment, the collection unit 151 may provide the collected images to the cloud-based AI-as-a-service (AI). At this point, the images may be collected by the collection unit 151 and provided to the artificial intelligence (AI) service as the collection unit 151 is connected to the network 400 through the control unit 160 and/or the communication unit 110, and connected to the cloud-based AI-as-a-service (AI).

The generation unit 152 may generate a result of diagnosing any one or more among the skin, scalp, health, and/or preference of the user on the basis of the survey response information and/or image.

The generation unit 152 may generate a result of diagnosing any one or more among the skin, scalp, health, and/or preference of the user using an analysis result provided by the artificial intelligence (AI) service. At this point, the image may be provided to the artificial intelligence (AI) service through the collection unit 151, the control unit 160, and/or the communication unit 110.

The generation unit 152 may recommend a recipe according to the generated diagnosis result. At this point, the recipe may be recommended on the basis of a diagnosis code stored in advance and a raw material code set to correspond to the diagnosis code according to the diagnosis result described below.

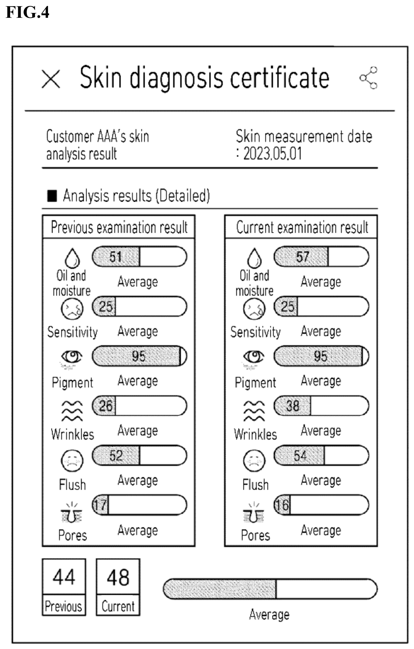

FIG. 4 is an exemplary view schematically showing an example of a skin diagnosis result of a customer according to an embodiment of the present invention.

Referring to FIG. 4, the generation unit 152 may generate a result of diagnosing skin items including oil and moisture, sensitivity, pigmentation, wrinkles, flush, and/or pores of the skin of the user as a skin diagnosis result on the basis of the survey response information and/or image.

The generation unit 152 may generate a result of diagnosing pigmentation on the basis of the image of the user. When the generation unit 152 detects a dark pigment area using the brightness value of the image and removes the red pigment using the value of color difference, only the melanin pigment may remain. The pigment is numerically calculated only as a brightness value, and the pigmentation may be calculated by primarily giving a weight value according to the degree of darkness, secondarily giving the degree of darkness compared to the surrounding areas as a weight value, adding the weight values, and dividing the added value by the magnitude value of the image.

The generation unit 152 may generate a result of diagnosing wrinkles on the basis of the image of the user. Since the wrinkles appear relatively dark compared to the surrounding areas like pores, the generation unit 152 may first detect dark areas from the image and then separately detect and remove hairs that appear similarly dark. Since the important factors of the wrinkles are the length and depth, the generation unit 152 may regard the areas with a length smaller than a predetermined value or a depth lower than the average as fine wrinkles. As the S channel of the HLS color has a value similar to an actual depth for the depth of the wrinkles, the generation unit 152 may calculate the length and depth of the wrinkles by using the S channel, multiplying the length and average depth values in each area finally determined as a wrinkle, and dividing the multiplied value by the magnitude value of the image.

The generation unit 152 may generate a result of diagnosing flush on the basis of the image of the user. Since the R color channel and/or the G color channel of the image are different from the surrounding areas, the generation unit 152 may calculate the flush by calculating as a combination of the difference between the R color channel and/or the G color channel and a constant value, and then dividing the calculated value by the magnitude value of the image.

The generation unit 152 may generate a result of diagnosing pores on the basis of the image of the user. Since pores appear relatively dark compared to the surrounding areas and have a round shape, the generation unit 152 may first detect dark areas in the image and then separately detect and remove hairs that appear similarly dark. The generation unit 152 may use a morphology technique of separating pores detected to be connected to each other as they are small or stretched, and as the size and depth of the pores are important, the numerical values of the pores may be calculated by multiplying the size and depth of each pixel for all pixels and dividing the value by the magnitude value of a second image.

As an optional embodiment, the diagnosis result of the pigmentation, wrinkles, flush, and/or pores may be generated on the basis of the image. However, for the oil and moisture and/or sensitivity, of which the diagnosis result is difficult to obtain only based on the image, the result of diagnosing oil and moisture and/or sensitivity may be generated using a moisture sensor, an oil sensor, a survey response result, and/or an analysis algorithm, which are not shown in the drawings, as well as the image. For example, the moisture sensor may be provided in the user terminal 200. In addition, the user-customized provision device 100 may provide oil paper, which may replace the oil sensor, to the user in advance.

In order to generate a result of diagnosing the moisture, the generation unit 152 may calculate the amount of moisture on the surface of the skin by contacting the moisture sensor on the surface of the skin and measuring the change in electrostatic capacity that varies according to distribution of moisture through a precision capacitor. Since the oil applied on the oil paper turns into a red color darker than the original red color of the paper, in order to generate an oil diagnosis result, the generation unit 152 may calculate the degree of oil using the color difference in the red area of the image captured by applying oil on the oil paper.

In order to calculate the sensitivity, the generation unit 152 may use a skin analysis algorithm that utilizes the survey response information and/or image of the user received in response to survey request information (e.g., Does your skin suddenly itch and sting when the external environment changes? Does a mark remain on your forehead when you press it with your fingernail? Does your skin sting or get troubles when you change cosmetics?, etc.). The skin analysis algorithm may utilize a method of diagnosing moisture and/or skin flush, and may convert the result of the skin analysis algorithm and/or survey response information into a score.

The generation unit 152 may recommend a product suitable for the user to the user. At this point, the recommendation can be made according to the following equation 1.

[Equation 1]

$$\frac{1}{3}\left[\left(\frac{F_p+P_p+W_p+C_p+W_p+O_p}{6} \times 0.56\right) + (PS \times 0.34) + (PSd \times 0.1)\right]$$

(Here, Fp: flush percentage, Pp: pore percentage, Wp: wrinkle percentage, Cp: pigment percentage, Op: oil and moisture percentage, Sp: sensitivity percentage, PS: purchase satisfaction survey value, PSd: big data label purchase satisfaction survey value)

The percentages of flush, pores, wrinkles, pigment, oil and moisture, and/or sensitivity in equation 1 are calculated using the flush, pores, wrinkles, pigment, oil and moisture, and/or sensitivity calculated above, a purchase satisfaction survey value is calculated by receiving answers to predetermined questions of a purchase satisfaction survey for a specific product from a customer terminal, and the big data label purchase satisfaction survey value may be calculated using a purchase satisfaction survey based on big data. In this way, custom-tailored recommendation information suitable for the customer may be provided to the customer using the values calculated through the formula of equation 1.

The generation unit 152 may generate a result of diagnosing scalp items including oil and moisture, sensitivity, hair thickness, hair damage, hair loss, and/or keratin for the scalp of the user as a scalp diagnosis result on the basis of the survey response information and/or image.

In order to generate a result of diagnosing scalp oil and moisture and/or sensitivity, the generation unit 152 may use the same method of generating a result of diagnosing skin oil and moisture and/or sensitivity.

The generation unit 152 may generate a result of diagnosing hair thickness on the basis of the scalp image of the user. The generation unit 152 may detect hair using the color difference after enlarging the scalp image of the user, and calculate thickness of the detected hair.

The generation unit 152 may generate a result of diagnosing hair damage on the basis of the survey response information and the scalp image of the user. The generation unit 152 may detect hair using the color difference after enlarging the scalp image of the user, and calculate a hair damage level using the roughness and color differences of the detected hair.

The generation unit 152 may generate a result of diagnosing hair loss on the basis of the survey response information and the scalp image of the user. The generation unit 152 may extract pores from the scalp image, count the number of hairs shown in the pores, and calculate density of the hair by counting the number of hairs per unit area. The result of diagnosing hair loss may be generated using the survey response information, the number of hairs per pore, density of the pore, thickness of the hair, and/or width of the forehead.

The generation unit 152 may generate a result of diagnosing keratin of the scalp on the basis of a scalp image of the user. The generation unit 152 may calculate the keratin level by detecting bright and/or dark areas in the scalp image compared to the surrounding areas, excluding the dark area as the hair area, and converting the bright area into a percentage.

Additionally, in order to generate a result of diagnosing scalp flush, the generation unit 152 may utilize the same method of generating a result of diagnosing skin flush. The degree of seborrhea (trouble) of the scalp may be calculated using the scalp flush, the keratin level of the scalp described above, and the survey response information.

As an optional embodiment, the generation unit 152 may use a known keratin measurement algorithm to calculate the keratin level of the scalp. The generation unit 152 may use the survey response information and/or an oil measurement algorithm to calculate the degree of oil of the scalp. The generation unit 152 may use the survey response information and/or moisture sensor information to calculate the degree of moisture of the scalp. The generation unit 152 may use the survey response information, a keratin algorithm, and a known flush algorithm to calculate the degree of sensitivity of the scalp. The generation unit 152 may use the survey response information, a keratin algorithm, and a flush algorithm to calculate the degree of seborrhea (trouble) of the scalp. The generation unit 152 may use the survey response information, an algorithm for counting the number of hairs per pore, an algorithm for calculating pore density, an algorithm for calculating hair thickness, and an algorithm for calculating the width of the forehead to calculate the degree of hair loss. The generation unit 152 may use the survey response information and an algorithm for calculating the thickness of hair to calculate the degree of hair damage.

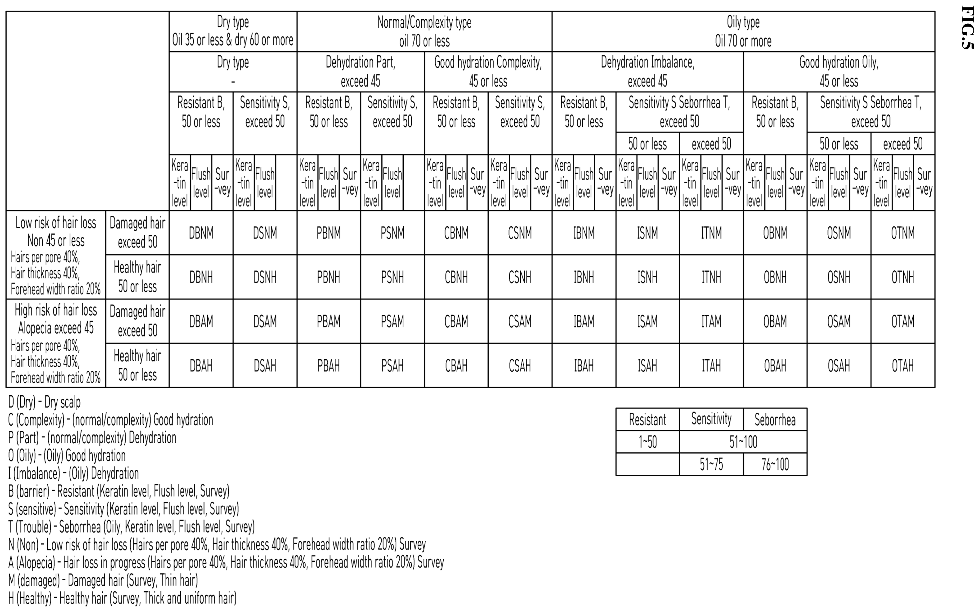

FIG. 5 is an exemplary view schematically showing an example of a table of scalp diagnosis criteria expressed in the form of MBTI for generating a scalp diagnosis result of a customer according to an embodiment of the present invention.

Referring to FIG. 5, the generation unit 152 may determine the scalp diagnosis result of a user as any one among a plurality of scalp types classified in the form of MBTI. FIG. 5 shows a table of scalp diagnosis criteria expressed in the form of MBTI for determining the scalp diagnosis result of the user. The table of scalp diagnosis criteria is stored in the database 140, and the scalp diagnosis result may be determined as any one type on the basis of the scalp diagnosis result of the user.

A plurality of scalp types classified in the form of MBTI may be classified into 48 scalp types on the basis of 12 criteria including dry scalp (D: dry), good hydration of normal scalp and/or complexity scalp (C: complexity), dehydration of normal scalp and/or complexity scalp (P: part), good hydration of oily scalp (O: oily), dehydration of oily scalp (I: imbalance), resistant scalp (B: barrier), sensitive scalp (S: sensitive), seborrheic scalp (T: trouble), low risk of hair loss (N: non), hair loss in progress (A: alopecia), damaged hair (M: damaged), and healthy hair (H: healthy).

For example, when the scalp type of the user is determined as PSAM, it can be seen that as the scalp of the user is a normal/complexity type, in which the scalp is dehydrated and sensitive and the damaged hair exceeds a criterion, the risk of hair loss is high. In addition, when the scalp type of the user is determined as OTNM, it can be seen that although the scalp of the user is oily, in which the moisture is sufficient, the sensitivity and/or seborrhea exceeds a criterion, and the damaged hair exceeds a criterion, the risk of hair loss is low.

The generation unit 152 may assign a diagnosis code according to the diagnosis result. In addition, a raw material code may be assigned to each diagnosis code to correspond to the diagnosis code.

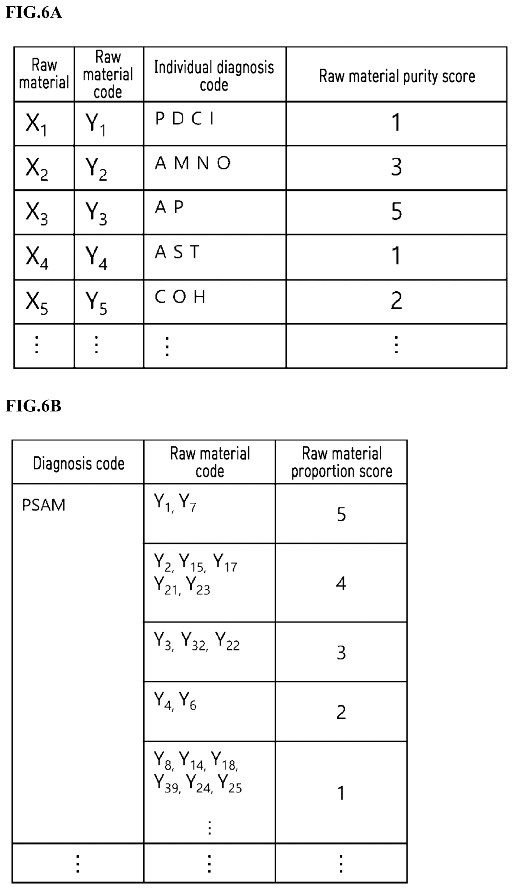

FIG. 6a and FIG. 6b are tables schematically showing an example of storing raw material information according to an embodiment of the present invention.

Referring to FIG. 6a, the generation unit 152 may assign a code to a raw material to correspond to a diagnosis code assigned on the basis of 12 criteria including dry scalp (D: dry), good hydration of normal scalp and/or complexity scalp (C: complexity), dehydration of normal scalp and/or complexity scalp (P: part), good hydration of oily scalp (O: oily), dehydration of oily scalp (I: imbalance), resistant scalp (B: barrier), sensitive scalp (S: sensitive), seborrheic scalp (T: trouble), low risk of hair loss (N: non), hair loss in progress (A: alopecia), damaged hair (M: damaged), and healthy hair (H: healthy).

As a specific example, each of the 12 criteria may configure a diagnosis code, and a raw material code corresponding to each diagnosis code may be stored in the diagnosis code in advance. Therefore, as shown in FIG. 6b, when the scalp type of a user is determined as PSAM, codes of corresponding raw materials are stored in advance in the diagnosis codes P, S, A, and M, respectively, and raw materials suitable for the PSAM can be recommended on the basis of the diagnosis codes. At this point, a recipe suitable for a diagnosis type may be recommended on the basis of the raw material purity score stored in advance for each raw material, the raw material proportion score stored to be suitable for each diagnosis type, and the like. For example, the raw material purity score and the raw material proportion score may be set on the basis of data obtained by analyzing products certified by the Food and Drug Administration (FDA) of the United States, the Ministry of Food and Drug Safety (MFDS), and the GMP in the country where the manufacturing facility is located, and/or artificial intelligence (AI). As a specific example, the raw material purity score and the raw material proportion score may be set on the basis of data obtained by analyzing raw materials of products certified by the FDA, the MFDS, and the GMP in the country where the manufacturing facility is located using artificial intelligence.

For example, as for the purity score of a raw material, a higher score may be given to a raw material that is closer to, for example, a natural or organic material without any other material mixed therein, and a lower score may be given to a synthetic material. In addition, as for the proportion score of a raw material, a higher score may be given a raw material that is more effective and suitable for a diagnosis type, and a gradually lower score may be given according the effect. For example, the score may be granted in a way of giving 5 points when the effect is very high, 4 points when the effect is high, 3 points when the effect is moderate, 2 points when the effect is low, 1 point when the effect is very low, and 0 point when the effect is ignorable. The raw material purity score and/or the raw material proportion score given for each raw material in this way may be used in recommending a diagnosis recipe. A recommendation score may be calculated by adding the raw material purity scores and the raw material proportion scores, and the calculated recommendation score may be provided to the user.

The generation unit 152 may determine a setting for mixing the raw materials recommended according to the diagnosis result based on the survey response information and/or image at an appropriate mixing ratio. At this point, the mixing ratio may be set on the basis of data obtained and stored in advance by analyzing raw materials of products certified by the FDA, the MFDS, and the GMP in the country where the manufacturing facility is located using artificial intelligence.

As an optional embodiment, the generation unit 152 may calculate the types, amounts, and the like of raw materials suitable for the user using the artificial intelligence AI learned to output a required amount of raw materials on the basis of the diagnosis result based on the survey response information and/or image, and set an appropriate mixing ratio suitable for the calculated type and/or amount of the raw materials.

The generation unit 152 may generate a result of diagnosing health items including the blood pressure, heart rate, electrocardiogram, stress index, fatigue, and body parts requiring treatment (nervous system, sensory system, digestive/metabolic system, cardiovascular system, endocrine system, reproductive system, urinary system, muscular system, immune system, etc.) of the user, and/or the types of required nutrients as a health diagnosis result on the basis of the survey response information and/or image.

In order to generate a result of diagnosing blood pressure, heart rate, and/or electrocardiogram, the generation unit 152 may use the survey response information and a sensors provided in the user terminal 200.

In order to generate a result of diagnosing a stress index and/or fatigue, the generation unit 152 may use the survey response information.

In order to generate a result of diagnosing body parts that require treatment, the generation unit 152 may select a body part (nervous system, sensory system, digestive/metabolic system, cardiovascular system, endocrine system, reproductive system, urinary system, muscular system, immune system, etc.) that fall outside a standard or normal range by comparing on the basis of the survey response information and health index reference standard data input in advance.

In order to generate a result of diagnosing nutrition facts required by the user, the generation unit 152 may select an excessive nutrient group and a deficient nutrient group on the basis of the survey response information, data on the daily recommended intake for each of the nutrition facts input in advance, and/or the result of diagnosing body parts that require treatment, and combine the nutrition facts to calculate the types and amounts of the required nutrients.

The generation unit 152 may generate a result of diagnosing preference items including any one or more among the taste, scent, nicotine, and caffeine selected differently according to the preference items (cigarettes, alcohol, coffee, etc.) or perfumes preferred by the user as a preference diagnosis result on the basis of the survey response information and/or image.

In order to generate a result of diagnosing the taste preferred by the user, the generation unit 152 may generate a result of diagnosing the taste of the user on the basis of the survey response information and data on pure tastes (sweet, salty, bitter, sour, savory, fatty, etc.), tastes by pain (spicy, astringent, etc.), and/or flavors (chocolate, vanilla, mint, etc.) input in advance.

In order to generate a result of diagnosing the type and/or concentration of a scent preferred by the user, the generation unit 152 may generate a result of diagnosing the type and/or concentration of the scent on the basis of data such as survey response information and fragrances input in advance, for example, edible fragrances (strawberry, peach, chocolate, vanilla, mint, etc.) and/or cosmetic fragrances (musk, aroma, fruity, woody, citrus, oriental, metallic, honey, green, dry, leathery, marine, minty, spicy, floral, powdery, watery, etc.). For the type and/or concentration of the scent, a result of diagnosing the type and/or concentration of the scent may be generated using a scent sensor and a survey response result. For example, the scent sensor may be provided in the customer terminal 200. In addition, the user-customized provision device 100 may provide a standard scent tester capable of comparing scent concentrations, which can replace the scent sensor, to the user in advance. At this point, the calculated scent diagnosis result may be used to recommend and/or manufacture perfumes, in addition to the preference items.

In order to generate a result of diagnosing the content of nicotine, the generation unit 152 may generate a result of diagnosing the content of nicotine by using the survey response information and data such as the usage and dosage of nicotine, and the content of nicotine contained in commercially available tobacco products input in advance.

In order to generate a result of diagnosing the content of caffeine, the generation unit 152 may generate a result of diagnosing the content of caffeine by using the survey response information and data such as the usage and amount of caffeine, and content of caffeine contained in commercially available coffee products input in advance.

For example, when the product preferred by the user is perfume, in order to generate a result of diagnosing the preference for the perfume, the generation unit 152 may generate a result of diagnosing preference items for fragrance among the preference items including taste, scent, nicotine, and caffeine. On the basis of the survey response information and the scent information input in advance, the generation unit 152 may determine a scent through an appropriate combination of scents selected according to the top note, middle note, and base note of perfumes, and calculate the concentration of each selected scent using a scent sensor provided in the user terminal 200 or a standard scent tester.

In addition, when the product preferred by the user is cigarette, in order to generate a result of diagnosing the preference for the cigarette, the generation unit 152 may generate a result of diagnosing preference items for the scent and nicotine among the preference items including taste, scent, nicotine, and caffeine. The generation unit 152 may generate a result of diagnosing the scent and nicotine content of cigarettes on the basis of the survey response information and information on the scent and nicotine input in advance.

The provision unit 153 may provide one or more of the diagnosis results described above and solution information to the user terminal 200, and recommend purchase of products that assist execution of the solution. In addition, the recommended products may be provided as QR codes on the screen of the user terminal 200. The QR codes may be transmitted to the product manufacturing device 300 in response to the product purchase request of a customer.

The diagnosis results of the user, solution, and/or product purchase recommendation information provided by the provision unit 153 may be provided to the user terminal 200 in various methods.

The provision unit 153 may provide the diagnosis results of the user to the user terminal 200 in a numerical form. For example, the provision unit 153 may provide numerical values according to the diagnosis results as a chart or provide the diagnosis result items of the user in the shape of bars. As the diagnosis result items are provided in the shape of bars, the user may intuitively grasp the diagnosis result items. In addition, as the values of previous diagnosis results and/or the values of the current diagnosis result are provided together, the user may compare the diagnosis results.

The provision unit 153 may provide comments on the current state according to the diagnosis result of the user to the user terminal 200, and provide comprehensive values according to the diagnosis result. The comprehensive values may be provided as, for example, skin age, health age, or the like.

The provision unit 153 may provide a primary solution to the user terminal 200. For example, with respect to the diagnosis result of the user, the provision unit 153 may provide previous and/or current images or scores, and comments thereon.

The provision unit 153 may provide a secondary solution to the user terminal 200. For example, the provision unit 153 may provide product recipe information for manufacturing a user-customized product corresponding to the diagnosis result, and comments on the result of analyzing the current diagnosis.

As an optional example, comments on the skin care, methods for skin massage, and images compared before and after the skin massage performed using the methods may be provided. Furthermore, information on the parts that require intensive care may be provided so that the previous and/or current states may be compared. In addition, comments on health care and methods for taking health functional foods, for example, methods of preventing side effects, misuse, and reduction in the effect through comparison of ingredients of currently taking medicines or health functional foods with ingredients of recommended health functional foods, may be provided.

The provision unit 153 may provide information on the history of the diagnosis items to the user terminal 200 in a graphical form. For example, the results of first to seventh diagnostic tests conducted previously may be provided in a graphical form, together with the result of the eighth diagnostic test conducted at this time. As the history information is used in this way, the user may intuitively confirm changes of his or her own.

The provision unit 153 may provide a tertiary solution to the user terminal 200. For example, the provision unit 153 may provide a program recommended for treatment, an application menu for receiving non-face-to-face coaching service of experts, and a manufacturing menu for manufacturing and/or purchasing custom-tailored skin products using recipe information corresponding to the diagnosis result. When the manufacturing menu is selected, a QR code may be transmitted to the product manufacturing device 300.

The provision unit 153 may provide product recipe information to manufacture and/or purchase user-customized products.

As an optional embodiment, the provision unit 153 may provide recipe information so that the user may purchase user-customized skin products and/or scalp products. For example, the products may include one or more among an oily light type product quickly absorbed into the skin with a light dosage form, a universal medium type product suitable for both oily and dry skins, a dry heavy type product wrapping the surface of the skin for a long time, a heavy type product wrapping the surface of the epidermis for a long time, a light type product quickly absorbed into the skin due to a light dosage form, a lifting product improving wrinkles on the skin with a herbal complex containing patented anti-wrinkle ingredients, a brightening product suppressing melanin synthesis with plant-based ingredients containing whitening ingredients, a moisturizing product having high moisturizing and moisture retention with a moisturizing ingredient of hyaluronic acid, an elasticity product creating elasticity components of the skin and/or enhancing elasticity using human collagen, a trouble-solving product improving skin condition with patented antibacterial and anti-acne ingredients, a soothing product providing moisturizing and/or skin soothing effects with six herbs, a sebum control product that controls the amount of sebum by adjusting the oil and moisture balance of the skin, and a pore product that prevents pore expansion by stabilizing the production cycle of the stratum corneum.

As an optional embodiment, the provision unit 153 may provide recipe information so that the user may purchase user-customized health functional foods. For example, the functional foods may include any one or more among a nutritional supplement product containing vitamins and/or minerals, such as vitamin A, beta-carotene, vitamin D, vitamin E, vitamin K, vitamin B1, vitamin B2, niacin, pantothenic acid, vitamin B6, folic acid, vitamin B12, biotin, vitamin C, calcium, magnesium, iron, zinc, copper, selenium (selenium), iodine, manganese, molybdenum, potassium, chromium, and the like, a product containing sugars and/or carbohydrates, such as glucosamine, N-acetylglucosamine, mucopolysaccharide protein, dietary fiber, aloe gel, reishi mushroom fruiting body extract, chitosan, chitooligosaccharides, and/or fructooligosaccharides, a product containing terpenes such as ginseng, red ginseng, chlorophyll, spirulina, and/or chlorella, a product containing phenols such as aloe whole leaves, green tea extract, and/or propolis extract, a product containing pollens, a product containing fatty acids and/or lipids, such as squalene, omega-3 fatty acid containing oil, gamma-linolenic acid containing oil, lecithin, squalene, phytosterols, phytosterol esters, alkoxyglycerols containing shark liver oil, octacosanol containing oil, and/or plum extracts, a product containing enzymes, a product containing chitosan, a product containing fermented microorganisms, such as lactic acid bacteria, probiotics, and/or red yeast rice, a product containing amino acids and/or proteins, a product containing royal jelly, a product containing mushrooms and/or plant extracts, and materials containing yeast. The health functional foods may include various dosage forms, and may be provided in one or more forms selected from, for example, capsule, tablet, powder, liquid, granule, and/or jelly.

As an optional embodiment, the provision unit 153 may provide recipe information so that the user may purchase user-customized preference items or perfume products. For example, the preference items or perfume products may include any one or more among tobacco products including cigarettes, kreteks, bidis, sticks, cigars, e-cigarettes, liquid e-cigarettes, pipe tobacco, hookah, dried snuff, inhaled tobacco, dissolving tobacco, and chewing tobacco, perfume products including perfumes, eau de perfumes, eau de toilette, and/or eau de cologne containing any one or more selected from musk scent oil, aromatic scent oil, fruity scent oil, woody scent oil, citrus scent oil, oriental scent oil, metallic scent oil, honey scent oil, green scent oil, dry scent oil, leathery scent oil, marine scent oil, minty scent oil, spicy scent oil, floral scent oil, powdery scent oil, and watery scent oil, and coffee products including Arabica beans, Robusta beans and/or Liberica beans.

In this embodiment, the raw materials of the product included in the recipe information are not fixed and may be changed, and the product may be optimized while changing the raw materials.

The provision unit 153 may provide a non-face-to-face coaching service to the user terminal 200 for one or more of the diagnosis results described above. When selection of a menu item for requesting a non-face-to-face coaching service is received from the user terminal 200, the provision unit 153 may provide the non-face-to-face coaching service. The provision unit 153 may match an expert with each user to provide the non-face-to-face coaching service. For example, information on the profile of various experts may be stored in the database. The provider may select an expert corresponding to a result of diagnosing any one or more among the skin, scalp, health, and/or preference of the user, and match the user with the expert. In another embodiment, the user himself or herself may view the profile information of the experts and select an expert to receive the coaching service.

As an optional embodiment, the provision unit 153 may acquire, from the terminal of the matched expert, one or more of the skin diagnosis results and/or scalp diagnosis results generated by the expert, product recipe information based on one or more of the skin diagnosis results and/or scalp diagnosis results, and coaching data based on manufactured customized cosmetics, skin history information, and/or skin care information, and provide the data to the user terminal 200. In this process, a 1:1 non-face-to-face consultation may be performed between the user and the expert. The provision unit 153 may provide the coaching data generated by the expert to the user terminal 200 at a predetermined time and/or cycle.

As an optional embodiment, the provision unit 153 may acquire, from the terminal of the matched expert, the health diagnosis result generated by the expert, product recipe information based on the health diagnosis result, and coaching data based on manufactured customized health functional foods, health history information, and/or health care information, and provide the data to the user terminal 200. In this process, a 1:1 non-face-to-face consultation may be performed between the user and the expert. The provision unit 153 may provide the coaching data generated by the expert to the user terminal 200 at a predetermined time and/or cycle.

As an optional embodiment, the provision unit 153 may acquire, from the terminal of the matched expert, the preference diagnosis result generated by the expert, product recipe information based on the preference diagnosis result, and coaching data based on history information and/or treatment information of customized preference items or perfumes that have been manufactured, and provide the data to the user terminal 200. In this process, a 1:1 non-face-to-face consultation may be performed between the user and the expert. The provision unit 153 may provide the coaching data generated by the expert to the user terminal 200 at a predetermined time and/or cycle.

As an optional embodiment, the provision unit 153 may provide a customized container label. For example, the label may include product information and user information, and may include an area other than the space containing the product information and user information. At this point, areas other than the space containing the product information and user information may be freely customized by the user using emoticons and/or image characters to have various designs. At this point, customization of the label may be provided to the user terminal 200 through the provision unit 153, and the user may easily design and customize the label displayed through the user terminal 200.

The processing unit 154 may transmit inspection request information to each of a plurality of product manufacturing devices 300 in response to a product purchase request signal from the user. Here, when the manufacturing menu of the user terminal 200 is selected, the processing unit 154 may receive the product purchase request signal from the user. In addition, the inspection request information may include raw material inventory request information, container inventory request information, state request information, and/or manufacturing environment request information of each product manufacturing device 300.

The processing unit 154 may receive inspection response information in response to the inspection request information from each of the plurality of product manufacturing devices 300. Here, the inspection response information may include raw material inventory response information, container inventory response information, state response information, and/or manufacturing environment response information. In addition, the state response information may include whether the product manufacturing device 300 is operating normally, the number of production orders, the number of manufactured products, the defect rate, the worker, the manufacturing progress location (e.g., container insertion, container separation, raw material discharge, container assembly, stirring, etc.), raw material discharge amount, raw material expiry date, raw material order state, raw material purchase and stocking state, and the like. In addition, the manufacturing environment response information may include the temperature and/or humidity inside the product manufacturing device 300, and the cleaning state inside thereof.

The processing unit 154 may select at least one product manufacturing device 300 capable of manufacturing a user-customized product on the basis of the inspection response information received from each of the plurality of product manufacturing devices 300, transmit user-customized product manufacturing information to the selected product manufacturing device 300, and instructs the product manufacturing device 300 to perform a task. Here, the user-customized product manufacturing information may include one or more among the recipe information, raw material mixing information, container information, and stirring information corresponding to any one or more selected from the skin diagnosis results, scalp diagnosis results, health diagnosis results, and/or preference diagnosis results of the user.

As a manufacturing completion signal for the user-customized product is received from the product manufacturing device 300, the processing unit 154 may request quality inspection of the user-customized product. Here, quality inspection of the user-customized product may be performed by the product manufacturing device 300. Alternatively, an external quality assessment company (not shown) may be requested to performed the quality inspection, through the product manufacturing device 300 that has received the request of the processing unit 154.

As a quality inspection completion signal for the user-customized product is received from the product manufacturing device 300, the processing unit 154 may request packaging of the user-customized product.

As a packaging completion signal for the user-customized product is received from the product manufacturing device, the processing unit 154 may transmit delivery information of the user to a delivery company (not shown) and request delivery of the user-customized product. The delivery company may pick up the user-customized product from the logistics warehouse of the product manufacturing device 300, and deliver the product to the user.

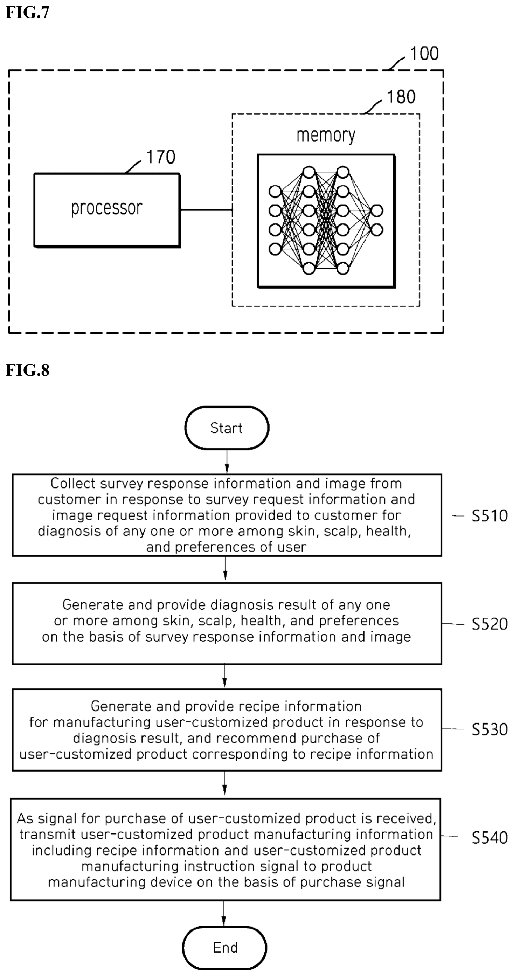

FIG. 7 is a block diagram schematically showing the configuration of a user-customized provision device 100 according to another embodiment. In the following description, any part overlapped with the descriptions of FIGS. 1 to 3 will be omitted. Referring to FIG. 7, the user-customized provision device 100 according to another embodiment may include a processor 170 and a memory 180.

In this embodiment, the processor 170 may process the functions performed by the communication unit 110, the storage medium 120, the program storage unit 130, the database 140, the service provision management unit 150, and/or the control unit 160 disclosed in FIGS. 2 and 3.

The processor 170 may control the entire operation of the user-customized provision device 100. Here, the term "processor" may mean, for example a data processing device embedded in hardware having physically structured circuits to perform the functions expressed as a code or instructions included in a program. An example of the data processing device embedded in hardware may be any one selected from processing devices such as a microprocessor, a central processing unit, a processor core, a multiprocessor, an ASIC, and an FPGA, but it is not limited thereto.

The memory 180 is operably connected to the processor 170 and may store at least one code in association with the operation performed by the processor 170.

In addition, the memory 180 may perform a function of temporarily or permanently storing data processed by the processor 170, and include data constructed as the database 140. Here, the memory 180 may include a magnetic storage medium or a flash storage medium, but the scope of the present invention is not limited thereto. The memory 180 may include a built-in memory and/or an external memory, and may include a volatile memory such as DRAM, SRAM, or SDRAM, a non-volatile memory such as OTPROM, PROM, EPROM, EEPROM, mask ROM, flash ROM, NAND flash memory, or NOR flash memory, a flash drive such as SSD, CF card, SD card, Micro-SD card, Mini-SD card, xD card, or Memory Stick, and a storage device such as HDD.

FIG. 8 is a flowchart illustrating a method for providing a user-customized product according to an embodiment of the present invention. In the following description, any part overlapped with the descriptions of FIGS. 1 to 7 will be omitted. The method for providing a user-customized product according to this embodiment will be described assuming that the user-customized provision device 100 is executed by the processor 170 with the assistance of peripheral components.

Referring to FIG. 8, at step S510, the processor 170 may transmit survey request information and/or image request information to the user terminal 200 connected through the network 400 to diagnose any one or more among the skin, scalp, health, and/or preference of the user, and collect survey response information and/or image from the user terminal 200 in response to the survey request information and/or image request information.

At step S520, the processor 170 may generate a result of diagnosing any one or more among the skin, scalp, health, and/or preference of the user on the basis of the survey response information and/or image, and provide the diagnosis result to the user terminal 200.

For example, the processor 170 may generate a result of diagnosing skin items including oil and moisture, sensitivity, pigmentation, wrinkles, flush, and/or pores of the skin of the user as a skin diagnosis result. In addition, the processor 170 may generate a result of diagnosing scalp items including oil and moisture, sensitivity, hair thickness, hair damage, hair loss, and/or keratin for the scalp of the user as a scalp diagnosis result. The processor 170 may determine the scalp diagnosis result as any one among a plurality of scalp types classified in the form of MBTI. Here, the plurality of scalp types classified in the form of MBTI may be classified into 48 scalp types on the basis of 12 criteria including dry scalp (D: dry), good hydration of normal scalp and/or complexity scalp (C: complexity), dehydration of normal scalp and/or complexity scalp (P: part), good hydration of oily scalp (O: oily), dehydration of oily scalp (I: imbalance), resistant scalp (B: barrier), sensitive scalp (S: sensitive), seborrheic scalp (T: trouble), low risk of hair loss (N: non), hair loss in progress (A: alopecia), damaged hair (M: damaged), and healthy hair (H: healthy). In addition, the processor 170 may generate a result of diagnosing health items including the blood pressure, heart rate, electrocardiogram, stress index, fatigue, and body parts requiring treatment (nervous system, sensory system, digestive/metabolic system, cardiovascular system, endocrine system, reproductive system, urinary system, muscular system, immune system, etc.) of the user, and/or the types of required nutrients as a health diagnosis result, and generate a result of diagnosing preference items including any one or more among the taste, scent, nicotine, and caffeine selected differently according to the preference items (cigarettes, alcohol, coffee, etc.) or perfumes preferred by the user as a preference diagnosis result.

At step S530, the processor 170 may generate recipe information for manufacturing a user-customized product in response to one or more among the skin diagnosis result, scalp diagnosis result, health diagnosis result, and preference diagnosis result, and provide the generated recipe information to the user terminal 200. In addition, the processor 170 may recommend purchase of a user-customized product corresponding to the recipe information.

As an optional embodiment, the recipe information may be recommended on the basis of a raw material code set in advance to correspond to each diagnosis code according to the diagnosis code assigned to a diagnosis result. As a specific example, the recipe information may be recommended in order of high score by the sum of the raw material purity score and the raw material proportion score, on the basis of the raw material purity score stored in advance for each raw material and the raw material proportion score stored in advance for each diagnosis type.

For example, the raw material purity score and the raw material proportion score may be set on the basis of data obtained by analyzing products certified by the U.S. Food and Drug Administration (FDA), the MFDS, and the GMP in the country where the manufacturing facility is located, and/or artificial intelligence (AI). As a specific example, the raw material purity score and the raw material proportion score may be set on the basis of data obtained by analyzing raw materials of products certified by the FDA, the MFDS, and the GMP in the country where the manufacturing facility is located, and training the AI on the basis of the analyzed raw material data.

For example, as for the purity score of a raw material, a higher score may be given to a raw material that is closer to, for example, a natural or organic material without any other material mixed therein, and a lower score may be given to a synthetic material. In addition, as for the proportion score of a raw material, a higher score may be given a raw material that is more effective and suitable for a diagnosis type, and a gradually lower score may be given according the effect. For example, the score may be granted in a way of giving 5 points when the effect is very high, 4 points when the effect is high, 3 points when the effect is moderate, 2 points when the effect is low, 1 point when the effect is very low, and 0 point when the effect is ignorable. The raw material purity score and/or the raw material proportion score given for each raw material in this way may be used in providing a diagnosis recipe. A recommendation score may be calculated by adding the raw material purity scores and the raw material proportion scores, and the calculated recommendation score may be provided to the user.

Meanwhile, the raw materials of products certified by the FDA, the MFDS, and the GMP in the country where the manufacturing facility is located are analyzed, and the artificial intelligence learned based on the data of the analyzed raw materials may calculate a raw material mixing ratio corresponding to the diagnosis information, provide a recipe, and recommend user-customized products to the user according to the recipe.

As an optional embodiment, step S530 may further include a step of recommending purchase of a user-customized product corresponding to the recipe information.

In the method of recommending purchase of a user-customized product corresponding to the recipe information, a recommended product may be determined by equation 1 shown below.

[Equation 1]

$$\frac{1}{3}\left[\left(\frac{F_p+P_p+W_p+C_p+W_p+O_p}{6} \times 0.56\right) + (PS \times 0.34) + (PSd \times 0.1)\right]$$

(Here, Fp: flush percentage, Pp: pore percentage, Wp: wrinkle percentage, Cp: pigment percentage, Op: oil and moisture percentage, Sp: sensitivity percentage, PS: purchase satisfaction survey value, PSd: big data label purchase satisfaction survey value)

The percentages of flush, pores, wrinkles, pigment, oil and moisture, and/or sensitivity in equation 1 are calculated using the flush, pores, wrinkles, pigment, oil and moisture, and/or sensitivity calculated above, a purchase satisfaction survey value is calculated by receiving answers to predetermined questions of a purchase satisfaction survey for a specific product from a customer terminal, and the big data label purchase satisfaction survey value may be calculated using a purchase satisfaction survey based on big data. At this point, the flush, pores, wrinkles, pigment, oil and moisture, and/or sensitivity are major indexes for generating a diagnosis result, and the result value calculated by equation 1 may be provided in connection with the recipe information recommended by the diagnosis code and raw material code. In this way, recommended products can be provided to the customer using the value calculated through the formula of equation 1.

At step S540, as a signal for purchase of the user-customized product is received, the processor 170 may transmit user-customized product manufacturing information including recipe information and/or a manufacturing instruction signal for user-customized cosmetics to the product manufacturing device 300.

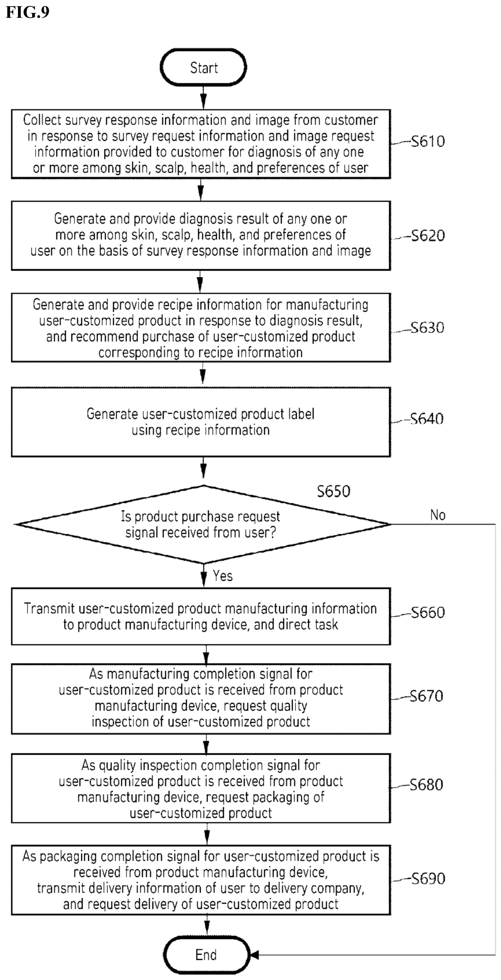

FIG. 9 is a flowchart illustrating a method for providing a user-customized product according to another embodiment of the present invention. In the following description, any part overlapped with the descriptions of FIGS. 1 to 8 will be omitted. The method for providing a user-customized service according to this embodiment will be described assuming that the user-customized provision device 100 is executed by the processor 170 with the assistance of peripheral components.

Referring to FIG. 9, at step S610, the processor 170 may transmit survey request information and/or image request information to the user terminal 200 connected through the network 400 to diagnose any one or more among the skin, scalp, health, and/or preference of the user, and collect survey response information and/or image from the user terminal 200 in response to the survey request information and/or image request information.

At step S620, the processor 170 may generate a result of diagnosing any one or more among the skin, scalp, health, and/or preference of the user on the basis of the survey response information and/or image, and provide the diagnosis result to the user terminal 200.

At step S630, the processor 170 may generate recipe information for manufacturing a user-customized product in response to any one or more among the skin diagnosis result, scalp diagnosis result, health diagnosis result, and/or preference diagnosis result, and provide the generated recipe information to the user terminal 200. In addition, the processor 170 may recommend purchase of a user-customized product corresponding to the recipe information.

As an optional embodiment, the recipe information may be recommended on the basis of a raw material code set in advance to correspond to each diagnosis code according to the diagnosis code assigned to a diagnosis result. As a specific example, the recipe information may be recommended in order of high score by the sum of the raw material purity score and the raw material proportion score, on the basis of the raw material purity score stored in advance for each raw material and the raw material proportion score stored in advance for each diagnosis type.

For example, the raw material purity score and the raw material proportion score may be set on the basis of data obtained by analyzing products certified by the U.S. Food and Drug Administration (FDA), the MFDS, and the GMP in the country where the manufacturing facility is located, and/or artificial intelligence (AI). As a specific example, the raw material purity score and the raw material proportion score may be set on the basis of data obtained by analyzing raw materials of products certified by the FDA, the MFDS, and the GMP in the country where the manufacturing facility is located, and training the AI on the basis of the analyzed raw material data.

For example, as for the purity score of a raw material, a higher score may be given to a raw material that is closer to, for example, a natural or organic material without any other material mixed therein, and a lower score may be given to a synthetic material. In addition, as for the proportion score of a raw material, a higher score may be given a raw material that is more effective and suitable for a diagnosis type, and a gradually lower score may be given according the effect. For example, the score may be granted in a way of giving 5 points when the effect is very high, 4 points when the effect is high, 3 points when the effect is moderate, 2 points when the effect is low, 1 point when the effect is very low, and 0 point when the effect is ignorable. The raw material purity score and/or the raw material proportion score given for each raw material in this way may be used in providing a diagnosis recipe. A recommendation score may be calculated by adding the raw material purity scores and the raw material proportion scores, and the calculated recommendation score may be provided to the user.

Meanwhile, the raw materials of products certified by the FDA, the MFDS, and the GMP in the country where the manufacturing facility is located are analyzed, and the artificial intelligence learned based on the data of the analyzed raw materials may calculate a raw material mixing ratio corresponding to the diagnosis information, provide a recipe, and recommend user-customized products to the user according to the recipe.

As an optional embodiment, step S530 may further include a step of recommending purchase of a user-customized product corresponding to the recipe information.

In the method of recommending purchase of a user-customized product corresponding to the recipe information, a recommended product may be determined by equation 1 shown below.

[Equation 1]

$$\frac{1}{3}\left[\left(\frac{F_p+P_p+W_p+C_p+W_p+O_p}{6}\times 0.56\right)+(PS\times 0.34)+(PSd\times 0.1)\right]$$

(Here, Fp: flush percentage, Pp: pore percentage, Wp: wrinkle percentage, Cp: pigment percentage, Op: oil and moisture percentage, Sp: sensitivity percentage, PS: purchase satisfaction survey value, PSd: big data label purchase satisfaction survey value)

The percentages of flush, pores, wrinkles, pigment, oil and moisture, and/or sensitivity in equation 1 are calculated using the flush, pores, wrinkles, pigment, oil and moisture, and/or sensitivity calculated above, a purchase satisfaction survey value is calculated by receiving answers to predetermined questions of a purchase satisfaction survey for a specific product from a customer terminal, and the big data label purchase satisfaction survey value may be calculated using a purchase satisfaction survey based on big data. At this point, the flush, pores, wrinkles, pigment, oil and moisture, and/or sensitivity are major indexes for generating a diagnosis result, and the result value calculated by equation 1 may be provided in connection with the recipe information recommended by the diagnosis code and raw material code. In this way, recommended products can be provided to the customer using the value calculated through the formula of equation 1.

At step S640, the processor 170 may provide label information of the user-customized product to the user terminal 200 connected through the network 400. At this point, a space where product information and/or user's order information are displayed may be provided on the label, and the areas other than the space where product information and/or user's order information are displayed may be freely customized by the user using emoticons and/or image characters to have various designs.

At step S650, the processor 170 may determine whether a product purchase request signal has been received from the user terminal 200.

At step S660, as a product purchase request signal is received from the user terminal 200, the processor 170 may transmit user-customized product manufacturing information including recipe information to the product manufacturing device 300 and direct a task. Here, before instructing the product manufacturing device 300 to perform a task, the processor 170 may transmit inspection request information to each of the plurality of product manufacturing devices 300. The processor 170 may receive inspection response information from each of the plurality of product manufacturing devices 301, 302, 303 in response to the inspection request information. The processor 170 may select at least one product manufacturing device 300 capable of manufacturing a user-customized product on the basis of the inspection response information.

At step S670, as a manufacturing completion signal for the user-customized product is received from the product manufacturing device 300, the processor 170 may request quality inspection of the user-customized product.

At step S680, as a quality inspection completion signal for the user-customized product is received from the product manufacturing device 300, the processor 170 may request packaging of the user-customized product.

As a packaging completion signal for the user-customized product is received from the product manufacturing device 300, the processor 170 may transmit delivery information of the user to a delivery company (not shown) and request delivery of the user-customized product.

The embodiments of the present invention described above may be implemented in the form of a computer program that can be executed through various components on a computer, and such a computer program may be recorded in a computer-readable medium. At this point, the medium may include a magnetic medium such as a hard disk, a floppy disk, and/or a magnetic tape, an optical recording medium such as a CD-ROM and/or a DVD, a magneto-optical medium such as a floptical disk, and/or a hardware device specially configured to store and execute program instructions, such as a ROM, a RAM, a flash memory, etc.

Meanwhile, the computer program may be specially designed and constructed for the present invention, or may be known and available to those skilled in the field of computer software. Examples of the computer program may include high-level language code that can be executed by a computer using an interpreter or the like, as well as machine language code such as those generated by a compiler.

The term "above" and/or directing terms similar thereto in the specification of the present invention (especially in the claims) may relate to both the singular and/or plural forms. In addition, when a range is described in the present invention, it includes inventions that apply individual values falling within the range (unless otherwise stated), and is equivalent to describing each individual value constituting the range in the detailed description of the invention.

Unless the sequence of the steps constituting the method according to the present invention are clearly described or there is no description contrary thereto, the steps may be executed in an appropriate order. The present invention is not necessarily limited to the order of describing the steps. All examples or exemplary terms (e.g., etc.) are used in the present invention only to describe the present invention in detail, and the scope of the present invention is not limited by the examples or exemplary terms unless otherwise limited by the claims. In addition, those skilled in the art will know that it can be configured according to the design conditions and/or factors within the scope of the patent claims having various modifications, combinations, and/or changes added thereto, or the equivalents thereof.

Therefore, the spirit of the present invention should not be defined to be limited to the embodiments described above, and all the scopes equivalent to the patent claims or equivalently modified therefrom, as well as the scope of the patent claims described below, are considered to fall within the scope of the present invention.

## Claims

1. A method for providing a customized product, the method comprising the steps of:

   collecting, from a user, survey response information and an image in response to survey request information and image request information provided to the user in order to diagnose any one or more among the skin, scalp, health condition, and preference of the user;

   generating a result of diagnosing any one or more among the skin, scalp, health condition, and preference of the user on the basis of the survey response information and the image;

   generating and providing recipe information for manufacturing a user-customized product with respect to the diagnosis result; and

   transmitting, to a product manufacturing device, a user-customized product manufacturing instruction signal including the recipe information on the basis of a user-customized product purchase signal received from the user, wherein

   the recipe information includes a raw material recommended by a diagnosis code including the diagnosis result and a raw material code including raw material information.

2. The method according to claim 1, wherein the raw material code further includes a raw material purity score and a raw material proportion score, and the recipe information provides a score calculated by a sum of the raw material purity score and the raw material proportion score.

3. The method according to claim 1, wherein the recipe information includes a raw material mixing ratio, wherein the raw material mixing ratio is calculated using artificial intelligence that has learned to output an amount of required raw materials.

4. The method according to claim 1, wherein the step of generating and providing recipe information for manufacturing a user-customized product with respect to the diagnosis result further includes the step of recommending purchase of a user-customized product corresponding to the recipe information, where a product recommended at the step of recommending purchase of a user-customized product is determined by equation 1.

[Equation 1]

$$\frac{1}{3}\left[\left(\frac{F_p + P_p + W_p + C_p + W_p + O_p}{6} \times 0.56\right) + (PS \times 0.34) + (PSd \times 0.1)\right]$$

(Here, Fp: flush percentage, Pp: pore percentage, Wp: wrinkle percentage, Cp: pigment percentage, Op: oil and moisture percentage, Sp: sensitivity percentage, PS: purchase satisfaction survey value, PSd: big data label purchase satisfaction survey value)

5. The method according to claim 1, wherein the image is transmitted to an artificial intelligence service, and the diagnosis result is generated using a result generated by analysis of the artificial intelligence.

6. The method according to claim 1, further comprising, after the step of generating and providing recipe information, the step of generating a user-customized product label using the recipe information, wherein a space where product information and user's order information are displayed may be provided on the label, and areas other than the space where product information and user's order information are displayed are freely customized by the user.

**FIG.1**

**FIG.2**

**FIG.3**

**FIG.4**

FIG.5

| | | Dry type Oil 35 or less & dry 60 or more | | Normal/Complexity type oil 70 or less | | | | Oily type Oil 70 or more | | | | | | |
|---|---|---|---|---|---|---|---|---|---|---|---|---|---|---|
| | | Dry type - | | Dehydration Part, exceed 45 | | Good hydration Complexity, 45 or less | | Dehydration Imbalance, exceed 45 | | | Good hydration Oily, 45 or less | | | |
| | | Resistant B, 50 or less | Sensitivity S, exceed 50 | Resistant B, 50 or less | Sensitivity S, exceed 50 | Resistant B, 50 or less | Sensitivity S, exceed 50 | Resistant B, 50 or less | Sensitivity S Seborrhea T, exceed 50 | | Resistant B, 50 or less | Sensitivity S Seborrhea T, exceed 50 | |
| | | | | | | | | | 50 or less | exceed 50 | | 50 or less | exceed 50 |
| | | Keratin level / Flush level / Survey | Keratin level / Flush level | Keratin level / Flush level / Survey | Keratin level / Flush level | Keratin level / Flush level / Survey | Keratin level / Flush level / Survey | Keratin level / Flush level / Survey | Keratin level / Flush level / Survey | Keratin level / Flush level / Survey | Keratin level / Flush level / Survey | Keratin level / Flush level / Survey | Keratin level / Flush level / Survey |
| Low risk of hair loss Non 45 or less — Hairs per pore 40%, Hair thickness 40%, Forehead width ratio 20% | Damaged hair exceed 50 | DBNM | DSNM | PBNM | PSNM | CBNM | CSNM | IBNM | ISNM | ITNM | OBNM | OSNM | OTNM |
| | Healthy hair 50 or less | DBNH | DSNH | PBNH | PSNH | CBNH | CSNH | IBNH | ISNH | ITNH | OBNH | OSNH | OTNH |
| High risk of hair loss Alopecia exceed 45 — Hairs per pore 40%, Hair thickness 40%, Forehead width ratio 20% | Damaged hair exceed 50 | DBAM | DSAM | PBAM | PSAM | CBAM | CSAM | IBAM | ISAM | ITAM | OBAM | OSAM | OTAM |
| | Healthy hair 50 or less | DBAH | DSAH | PBAH | PSAH | CBAH | CSAH | IBAH | ISAH | ITAH | OBAH | OSAH | OTAH |

D (Dry) - Dry scalp
C (Complexity) - (normal/complexity) Good hydration
P (Part) - (normal/complexity) Dehydration
O (Oily) - (Oily) Good hydration
I (Imbalance) - (Oily) Dehydration
B (barrier) - Resistant (Keratin level, Flush level, Survey)
S (sensitive) - Sensitivity (Keratin level, Flush level, Survey)
T (Trouble) - Seborrhea (Oily, Keratin level, Flush level, Survey)
N (Non) - Low risk of hair loss (Hairs per pore 40%, Hair thickness 40%, Forehead width ratio 20%) Survey
A (Alopecia) - Hair loss in progress (Hairs per pore 40%, Hair thickness 40%, Forehead width ratio 20%) Survey
M (damaged) - Damaged hair (Survey, Thin hair)
H (Healthy) - Healthy hair (Survey, Thick and uniform hair)

| Resistant | Sensitivity | Seborrhea |
|---|---|---|
| 1~50 | 51~100 | |
| | 51~75 | 76~100 |

**FIG.6A**

| Raw material | Raw material code | Individual diagnosis code | Raw material purity score |
|---|---|---|---|
| $X_1$ | $Y_1$ | P D C I | 1 |
| $X_2$ | $Y_2$ | A M N O | 3 |
| $X_3$ | $Y_3$ | A P | 5 |
| $X_4$ | $Y_4$ | A S T | 1 |
| $X_5$ | $Y_5$ | C O H | 2 |
| ⋮ | ⋮ | ⋮ | ⋮ |

**FIG.6B**

| Diagnosis code | Raw material code | Raw material proportion score |
|---|---|---|
| PSAM | $Y_1, Y_7$ | 5 |
| | $Y_2, Y_{15}, Y_{17}$ $Y_{21}, Y_{23}$ | 4 |
| | $Y_3, Y_{32}, Y_{22}$ | 3 |
| | $Y_4, Y_6$ | 2 |
| | $Y_8, Y_{14}, Y_{18}$, $Y_{39}, Y_{24}, Y_{25}$ ⋮ | 1 |
| ⋮ | ⋮ | ⋮ |

**FIG.7**

**FIG.8**

Start

Collect survey response information and image from customer in response to survey request information and image request information provided to customer for diagnosis of any one or more among skin, scalp, health, and preferences of user — S510

Generate and provide diagnosis result of any one or more among skin, scalp, health, and preferences on the basis of survey response information and image — S520

Generate and provide recipe information for manufacturing user-customized product in response to diagnosis result, and recommend purchase of user-customized product corresponding to recipe information — S530

As signal for purchase of user-customized product is received, transmit user-customized product manufacturing information including recipe information and user-customized product manufacturing instruction signal to product manufacturing device on the basis of purchase signal — S540

End

**FIG.9**

Start

Collect survey response information and image from customer in response to survey request information and image request information provided to customer for diagnosis of any one or more among skin, scalp, health, and preferences of user —— S610

Generate and provide diagnosis result of any one or more among skin, scalp, health, and preferences of user on the basis of survey response information and image —— S620

Generate and provide recipe information for manufacturing user-customized product in response to diagnosis result, and recommend purchase of user-customized product corresponding to recipe information —— S630

Generate user-customized product label using recipe information —— S640

S650

Is product purchase request signal received from user? —— No

Yes

Transmit user-customized product manufacturing information to product manufacturing device, and direct task —— S660

As manufacturing completion signal for user-customized product is received from product manufacturing device, request quality inspection of user-customized product —— S670

As quality inspection completion signal for user-customized product is received from product manufacturing device, request packaging of user-customized product —— S680

As packaging completion signal for user-customized product is received from product manufacturing device, transmit delivery information of user to delivery company, and request delivery of user-customized product —— S690

End

**EP 4 712 004 A1**

## INTERNATIONAL SEARCH REPORT

| International application No. |
|---|
| **PCT/KR2024/006295** |

| A. | CLASSIFICATION OF SUBJECT MATTER |
|---|---|

**G06Q 30/06**(2012.01)i; **G06Q 50/04**(2012.01)i; **G06Q 30/02**(2012.01)i; **G16H 10/20**(2018.01)i; **A61B 5/00**(2006.01)i

According to International Patent Classification (IPC) or to both national classification and IPC

| B. | FIELDS SEARCHED |
|---|---|

Minimum documentation searched (classification system followed by classification symbols)

G06Q 30/06(2012.01); A45D 44/00(2006.01); A61B 5/00(2006.01); G06Q 30/02(2012.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 진단(diagnosis), 설문(questionnaire), 이미지(image), 맞춤형(customized), 제품(product), 레시피(recipe), 제조(manufacturing)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | KR 10-2022-0051830 A (LILLYCOVER CO., LTD.) 26 April 2022 (2022-04-26) See paragraphs [0040]-[0041], [0044]-[0049], [0062] and [0099] and claims 1 and 4. | 1-3,5-6 |
| A | | 4 |
| Y | KR 10-2022-0064418 A (LG H&H CO., LTD.) 18 May 2022 (2022-05-18) See paragraphs [0023], [0053] and [0135]-[0136] and claims 1 and 5. | 1-3,5-6 |
| Y | KR 10-2199440 B1 (MOIBEC INC.) 06 January 2021 (2021-01-06) See claim 1. | 2 |
| A | KR 10-2022-0081446 A (JANG, Eun Jin) 16 June 2022 (2022-06-16) See entire document. | 1-6 |
| A | KR 10-2022-0119958 A (PARK, Hyeun) 30 August 2022 (2022-08-30) See entire document. | 1-6 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **06 August 2024** | **07 August 2024** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

30

## INTERNATIONAL SEARCH REPORT
### Information on patent family members

| Patent document cited in search report | | | Publication date (day/month/year) | Patent family member(s) | | | Publication date (day/month/year) |
|---|---|---|---|---|---|---|---|
| KR | 10-2022-0051830 | A | 26 April 2022 | KR | 10-2024-0008956 | A | 19 January 2024 |
| | | | | US | 2023-0108573 | A1 | 06 April 2023 |
| | | | | WO | 2022-086004 | A1 | 28 April 2022 |
| KR | 10-2022-0064418 | A | 18 May 2022 | CN | 110036403 | A | 19 July 2019 |
| | | | | EP | 3564892 | A1 | 06 November 2019 |
| | | | | JP | 2020-500611 | A | 16 January 2020 |
| | | | | JP | 2022-109314 | A | 27 July 2022 |
| | | | | KR | 10-2018-0064963 | A | 15 June 2018 |
| | | | | KR | 10-2019-0050832 | A | 13 May 2019 |
| | | | | KR | 10-2022-0064416 | A | 18 May 2022 |
| | | | | KR | 10-2022-0087567 | A | 24 June 2022 |
| | | | | KR | 10-2023-0101782 | A | 06 July 2023 |
| | | | | KR | 10-2023-0148859 | A | 25 October 2023 |
| | | | | KR | 10-2653851 | B1 | 03 April 2024 |
| | | | | US | 11742089 | B2 | 29 August 2023 |
| | | | | US | 2019-0295728 | A1 | 26 September 2019 |
| | | | | US | 2023-0282366 | A1 | 07 September 2023 |
| | | | | WO | 2018-101572 | A1 | 07 June 2018 |
| KR | 10-2199440 | B1 | 06 January 2021 | KR | 10-2175504 | B1 | 06 November 2020 |
| | | | | KR | 10-2199441 | B1 | 06 January 2021 |
| | | | | WO | 2021-045293 | A1 | 11 March 2021 |
| KR | 10-2022-0081446 | A | 16 June 2022 | KR | 10-2518076 | B1 | 05 April 2023 |
| KR | 10-2022-0119958 | A | 30 August 2022 | KR | 10-2638539 | B1 | 08 March 2024 |

Form PCT/ISA/210 (patent family annex) (July 2022)